# EUROPÄISCHE PATENTSCHRIFT

(11) **EP 1 086 685 B1**
(45) Veröffentlichungstag und Bekanntmachung des Hinweises auf die Patenterteilung: **26.03.2003**
(21) Anmeldenummer: 00118596.6
(22) Anmeldetag: 28.08.2000
(51) Int. Cl.: A61K 7/135

(54) **Zwei-komponenten-kit eine lösung und eine Emulsion enthaltend und verfahren zum entfärben von gefärbten Haaren**
Two component kit containing a solution and an emulsion and method for removing hair dye
Kit comprenant une solution et une émulsion et méthode pour la décoloration des cheveux teints

(30) Priorität: 24.09.1999 DE 19945877
(43) Veröffentlichungstag der Anmeldung: 28.03.2001
(73) Patentinhaber: KPSS-Kao Professional Salon Services GmbH, 64297 Darmstadt (DE)
(72) Erfinder: Lorenz, Heribert, 64401 Gross-Bieberau (DE); Eberling, Walter, 64560 Riedstadt-Crumstadt (DE)

(56) Entgegenhaltungen:
- DE-A- 2 646 435
- DE-C- 211 697
- US-A- 3 617 167
- US-A- 3 630 655
- US-A- 3 800 809
- US-A- 3 892 845

## Beschreibung

Die Erfindung betrifft ein Mittel sowie ein Verfahren zum Entfärben von gefärbten menschlichen Haaren, insbesondere solchen, die einer oxidativen Haarfärbung unterzogen wurden, unter Anwendung dieses Mittels.
Haarfärbungen erfreuen sich großer Beliebtheit.
Neben temporären bzw. semipermanten Färbungen werden dabei auch permanente Färbungen durchgeführt, die auf Basis von Farbstoffvorprodukten, die unmittelbar vor der Anwendung mit einem Oxidationsmittel, insbesondere einer Wasserstoffperoxid-Zusammensetzung, zu Farbstoffen entwickelt werden, erfolgen.

Zuweilen ergibt sich beim Verbraucher auch der Wunsch, eine Färbung vorzeitig zu entfernen, oder zu verändern bzw. eine mißglückte Färbung rückgängig zu machen. Hierfür gibt es sogenannte Entfärbe- oder Abziehmittel, die vorwiegend auf Basis von reduzierenden Verbindungen aufgebaut sind.

Für diesen Zweck wurden insbesondere Natriumdithionit, Hydrogensulfite, Formaldehydsulfoxylate wie Hydroxymethansulfinsäure oder auch Ascorbinsäure vorgeschlagen.

Bekannt ist auch ein Verfahren zum Entfärben von oxidativ gefärbten Haaren, wobei eine saure und eine alkalische, ein Reduktionsmittel, nämlich Hydroxymethansulfinsäure, enthaltende Lösung unmittelbar vor dem Aufbringen auf dem Haar vermischt werden, wobei eine Lösung mit einem sauren pH-Wert erhalten wird, die dann zur Entfernung der unerwünschten Haarfärbung dient.

Dabei ist eine hohe Reduktionsmittelkonzentration wünschenswert, die jedoch zu einer erheblichen Geruchsbelastung führt.

Die naheliegendste Lösung dieses Problems, der Zusatz eines Parfums zu der das Reduktionsmittel enthaltenden Lösung, läßt sich überraschenderweise nicht durchführen, da sich gezeigt hat, daß Parfumzusammensetzungen in dieser Lösung nicht stabil sind, sondern diese unter Bildung eines Niederschlags trüb werden.

Die Erfindung hat sich daher die Aufgabe gestellt, ein Verfahren zum Entfärben von gefärbten Haaren und eine entsprechende Zusammensetzung aufzufinden, das diese Probleme überwindet.

Dieses Mittel besteht darin, zwei bis zur Anwendung getrennt gehaltene Zusammensetzungen A und B zur Verfügung zu stellen, wobei die Zusammensetzung A mindestens ein Reduktionsmittel, vorzugsweise in einer Menge von etwa 5 bis 50 Gew.-%, in wäßriger Lösung enthält und einen alkalischen pH-Wert zwischen 7,5 und 12,5 aufweist, und die Zusammensetzung B eine wäßrige Öl-in-Wasser-Emulsion darstellt, die mindestens eine Parfumkomponente, mindestens eine fett- oder ölartige Komponente und mindestens einen Emulgator enthält und einen stark sauren pH-Wert zwischen 1 und 4 aufweist, wobei das nach dem Mischen beider Zusammensetzungen erhaltene Produkt einen pH-Wert von 2,5 bis 5,0 aufweist.

Die Erfindung betrifft weiterhin ein Verfahren zum Entfärben von gefärbten Haaren unter Anwendung eines durch Vermischen der oben definierten Zusammensetzungen A und B erhaltenen Produktes auf gefärbtem Haar.

Die bis zur Anwendung von der sauren Zusammensetzung B getrennt gehaltene alkalische Reduktionsmittelzusammensetzung A enthält als Reduktionsmittel vorzugsweise Hydroxymethansulfinsäure bzw. deren wasserlösliche Salze, vorzugsweise in einer Menge von etwa 10 bis etwa 40, insbesondere etwa 20 bis etwa 40 Gew.-%, berechnet auf die Gesamtmenge dieser Zusammensetzung.

Alternativ oder im Gemisch mit Hydroxymethansulfinsäure können jedoch auch andere Reduktionsmittel wie Natriumdithionit, Alkalihydrogensulfite oder auch Reduktone wie Ascorbinsäure und deren Salze eingesetzt werden.

Diese Zusammensetzung A weist vorzugsweise einen pH-Wert zwischen etwa 8 und 12, insbesondere etwa 8,5 bis 11, beispielsweise 9 bis 10, auf, und enthält vorzugsweise weitere Bestandteile wie Verdickungsmittel.

Die Menge und Art des Reduktionsmittels ist bestimmend für den exakten pH-Wert, der auch durch direkten Alkalizusatz justiert werden kann.

Geeignete Verdickungsmittel sind beispielsweise Cellulosederivate wie Hydroxyethyl-, Hydroxypropyl- oder Hydroxypropylmethylcellulose, Guar Gum und dessen Derivate, Xanthan Gum und dessen Derivate, etc., vorzugsweise, je nach gewünschter Viskosität, in einer Menge von etwa 0,1 bis etwa 2,5, insbesondere etwa 0,25 bis etwa 2, vorzugsweise etwa 0,5 bis 1,5 Gew.-% der Zusammensetzung A.

Zusammensetzung B, die eine saure Emulsion mit einem pH-Wert von 1 bis 4, insbesondere etwa 1,5 bis 3, darstellt, enthält neben mindestens einer vorzugsweise organischen Säure wie Citronensäure, Weinsäure, Milchsäure, Brenztraubensäure, Äpfelsäure, Gluconsäure, Bernsteinsäure, Glykolsäure oder Glyoxylsäure mindestens eine fett- oder ölartige Verbindung und mindestens eine oberflächenaktive Substanz als Emulgator sowie ein Parfum zur Herstellung einer Öl-in-Wasser-Emulsion.

Die Menge der eingesetzten Säure ist natürlich abhängig von dem gewünschten pH-Wert der Emulsion.

Die Emulsion B enthält mindestens eine fett- oder ölartige Verbindung, vorzugsweise in einer Menge von etwa 0,5 bis etwa 10, vorzugsweise etwa 1 bis 7,5, insbesondere etwa 1,5 bis 5 Gew.-%.

Geeignete fett- bzw. ölartige Stoffe sind beispielsweise C₁₀-C₂₄-Fettalkohole, insbesondere Laurylkohol, Myristylalkohol, Palmitylalkohol, Cetylalkohol, Oleylalkohol, Stearylalkohol, Behenylalkohol, Gemische aus diesen Alkoholen untereinander und auch weiteren, beispielsweise Cocosfettalkohol oder Cetylstearylalkohol.

Weitere geeignete fett- und ölartige Stoffe, zu denen auch Wachse zählen können, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline. Synthetische Öle und Wachse sind beispielsweise Silikonöle, Polyethylenglykole, etc.
Weitere geeignete hydrophobe Komponenten sind insbesondere Fettsäureester wie Isopropylmyristat, -palmitat, -stearat und -isostearat, Oleyloleat, Isocetylstearat, Hexyllaurat, Dibutyladipat, Dioctyladipat, Myristiylmyristat, Oleylerucat, Polyethylenglykol- und Polyglycerylfettsäureester wie PEG-7-glycerylcocoat, Cetylpalmitat, etc.

Die Emulsion B enthält weiterhin mindestens einen Emulgator, vorzugsweise in einer Menge von etwa 0,25 bis 7,5, insbesondere etwa 0,3 bis 5 Gew.-%.
Geeignete Emulgatoren sind aus dem Stand der Technik hinreichend bekannt, vgl. beispielsweise K. Schrader, Grundlagen und Rezepturen der Kosmetika, 2. Aufl. (1989), S. 387-524.

Geeignet sind dabei insbesondere anionaktive, nichtionische, amphotere und/oder zwitterionische Substanzen.

Es können natürlich auch Gemische aus verschiedenen oberflächenaktiven Substanzen eingesetzt werden.

Bevorzugt sind anionische Emulgatoren vom Sulfat-, Sulfonat-, Carboxylat- und Alkylphosphat-Typ, gegebenenfalls im Gemisch mit den bekannten nichtionischen und/oder amphoteren (zwitterionischen)Tensiden, beispielsweise die bekannten C₁₀-C₁₈-Alkylsulfate und die entsprechenden Ethersulfate, beispielsweise C₁₂-C₁₄-Alkylethersulfat, Laurylethersulfat, insbesondere mit 1 bis 4 Ethylenoxidgruppen im Molekül, weiterhin Monoglycerid(ether)sulfate, Fettsäureamidsulfate, die durch Ethoxylierung und anschließende Sulfatierung von Fettsäurealkanolamiden erhalten werden, und deren Alkalisalze, sowie Salze langkettiger Mono- und Dialkylphosphaten, die milde, hautverträgliche Detergentien darstellen, α-Olefinsulfate bzw. deren Salze, Alkalisalze von Sulfobernsteinsäurehalbestern, beispielsweise das Dinatriumsalz des Monooctylsulfosuccinats, Alkalisalze langkettiger Monoalkylethoxysulfosuccinate, Alkylpolyethercarbonsäuren, Alkylamidopolyethercarbonsäuren und C₈-C₂₀-Acylisethionate.

Geeignet sind auch C₈-C₂₂-Acylaminocarbonsäuren und deren wasserlösliche Salze wie N-Lauroylglutamat, insbesondere als Natriumsalz, N-Lauroylsarcosinat, N-C₁₂-C₁₈-Acylasparaginsäure, N-Myristoylsarcosinat, N-Oleoylsarcosinat, N-Lauroylmethylalanin, N-Lauroyllysin und N-Lauroylaminopropylglycin, vorzugsweise in Form ihrer wasserlöslichen Alkali- oder Ammonium-, insbesondere Natriumsalze.

Geeignete nichtionische Tenside sind C₈-C₁₈-Alkylpolyglucoside, Fettalkoholethoxylate, Sorbitanester wie PEG-Sorbitanester, Fettsäurepolyglykolester sowie langkettige Aminoxide und Fettsäurealkanolamide, vorzugsweise im Gemisch mit anionischen oberflächenaktiven Substanzen.

Geeignete amphotere bzw. zwitterionische Tenside sind ebenfalls bekannt, insbesondere Fettsäureamidoalkylbetaine, Sulfobetaine, z.B. Laurylhydroxysulfobetain, und langkettige Aminosäuren wie Cocoaminoacetat, Cocoaminopropionat, Cocoamphoacetat und -propionat, insbesondere in Form ihrer Alkalisalze.

Der Anteil des Parfums in der erfindungsgemäßen Emulsion B liegt vorzugsweise bei etwa 0,1 bis 1, insbesondere etwa 0,2 bis etwa 0,8 Gew.-%.

Als Parfums können als solche übliche Kompositionen verwendet werden; durch die erfindungsgemäße Anwendung als Emulsion sind sie stabilisiert, ohne daß es unbedingt der zusätzlichen Mitverwendung eines Lösungsvermittlers bedarf, kompatibel auch in der Gesamtzusammensetzung und überdecken bei der Anwendung den Geruch der eingesetzten Reduktionsmittel.

Durch die Verwendung der erfindungsgemäßen Zusammensetzung wird darüber hinaus auch eine zusätzliche Pflegewirkung am Haar erreicht.

Das durch das Vermischen der Zusammensetzung A und B erhaltene Produkt haftet besser am Haar und tropft nicht ab.

Die Zusammensetzungen A und insbesondere B können noch weitere Stoffe enthalten, insbesondere haarkonditionierende und haarpflegende Substanzen wie kationische, nichtionische, amphotere, zwitterionische und/oder anionische Polymere, Proteinhydrolysate, Silikonderivate wie Organopolysiloxane, Panthenol, Pflanzenextrakte, Lecithin, Feuchthaltemittel, Komplexbildner, etc.

Besonders geeignet ist ein Viskositätsbereich zwischen etwa 100 und 7500 mPa.s, insbesondere etwa 500 bis 5000 mPa.s, gemessen bei 20°C im Brookfield-Viskosimeter (Spindel Nr. 4 bei 20 rpm) für das nach dem Mischen erhaltene Endprodukt.

Obwohl die Anwendung des erfindungsgemäßen Mittels in erster Linie für das Entfärben von mit üblichen Oxidationsfärbemitteln permanent gefärbtem Haar bestimmt ist, kann damit selbstverständlich auch mit direktziehenden Farbstoffen semipermanent gefärbtes Haar entfärbt werden, gegebenenfalls unter Verwendung niedriger Konzentrationen an Reduktionsmittel.

Die die getrennt gehaltenen Zusammensetzungen A und B enthaltenden Behältnisse sind vorzugsweise als Set konfektioniert; sie können auch in bekannten Zweikammerbehältnissen untergebracht sein, deren Trennwand vor der Vermischung zerstört oder entfernt wird.
Derartige Zweikammerbehältnisse sind aus dem Stand der Technik vielfältig bekannt.

Die Durchführung des erfindungsgemäßen Verfahrens erfolgt wie beschrieben:

Auf oxidativ gefärbtes Haar, das entfärbt werden soll, wird, vorzugsweise nach Anfeuchten bzw. Vorwäsche, das durch Vermischen etwa gleicher Anteile einer alkalischen Reduktionsmittelzusammensetzung A und einer sauren Emulsion B erhaltene Gemisch mit einem pH-Wert von 2,5 bis 5,0 aufgebracht und nach etwa fünf- bis dreißig-, insbesondere zehn- bis zwanzigminütiger Einwirkung mit Wasser gut gespült, vorzugsweise shampooniert, und gegebenenfalls noch mit einer ein- bis sechs-, vorzugsweise zwei- bis dreiprozentigen wäßrigen Wasserstoffperoxid-Lösung für etwa 1 bis 3 Minuten nachbehandelt.

Der folgende Vergleichsversuch erläutert die Wirkungsweise und Vorteile der erfindungsgemäßen Zusammensetzung unter Anwendung des erfindungsgemäßen Verfahrens:
Eine **Lösung A**, enthaltend

| | |
|---|---|
| 22 (g) | Hydroxymethansulfinsäure, Natriumsalz |
| 1 | Hydroxyethylcellulose (Natrosol^{R}250MXR) |
| 77 | Wasser (pH: 9,8) |

wurde mit der gleichen Menge einer **Emulsion B**, bestehend aus

| | |
|---|---|
| 10,0 (g) | Citronensäure |
| 2,0 | Cetylstearylalkohol |
| 0,5 | Natriumlaurylsulfat |
| 0,1 | Panthenol |
| 0,3 | Parfum |
| 0,1 | Tetranatrium-EDTA |
| 87,0 | Wasser (pH: 1,7) |

gründlich vermischt.

Es wurde ein wohlriechendes Gemisch mit einem pH-Wert von 2,9 und einer Viskosität von ∼ 1100 mPa.s, gemessen bei 20°C im Brookfield-Viskosimeter (Spindel Nr. 4, 20rpm), erhalten.

Das Mittel ließ sich tropffrei auf nasses, rot gefärbtes Haar auftragen. Nach 15 Minuten Einwirkungsdauer wurde gewaschen, shampooniert und getrocknet, wobei die ursprüngliche dunkelblonde Haarfarbe wiederhergestellt war.

In einem Vergleichsversuch wurde eine Mischung aus gleichen Teilen der Lösung A wie oben beschrieben und einer Lösung B der folgenden Zusammensetzung hergestellt

| | |
|---|---|
| 10,0 (g) | Citronensäure |
| 0,5 | Natriumlaurylsulfat |
| 0,1 | Panthenol |
| 89,4 | Wasser (pH-Wert; 1,7) |

Die Mischung beider Lösungen wies einen stechenden Geruch auf und ließ sich wesentlich ungleichmäßiger auf rot gefärbtes, nasses Haar auftragen. Nach 15-minütiger Einwirkung wurde shampooniert, wobei eine vollständige Farbentfernung und die Wiederherstellung der dunkelblonden Naturfarbe erst nach einem weiteren Behandlungsgang mit 2,5%-iger wäßriger H₂O₂-Lösung erreicht werden konnte.

## Patentansprüche

1. Mittel zum Entfärben von gefärbten Haaren, bestehend aus zwei bis zur Anwendung räumlich voneinander getrennt gehaltenen Zusammensetzungen A und B, wobei die Zusammensetzung A mindestens ein Reduktionsmittel in einer wäßrigen Grundlage enthält und einen alkalischen pH-Wert zwischen 7,5 und 12,5 aufweist, und die Zusammensetzung B als wäßrige Emulsion vorliegt, die mindestens einen fett- oder ölartigen Stoff, mindestens einen wasserlöslichen Emulgator und mindestens eine Parfumkomponente enthält und einen sauren pH-Wert von 1 bis 4 aufweist, wobei beim Vermischen der gleichen Anteile beider Zusammensetzungen ein gebrauchsfertiges Produkt mit einem pH-Wert zwischen 2,5 und 5,0 erhalten wird.

2. Mittel nach Anspruch 1, **dadurch gekennzeichnet, daß** die Zusammensetzung A etwa 5 bis etwa 50 Gew.-% Hydroxymethansulfinsäure und/oder ein Alkalisalz desselben enthält.

3. Mittel nach Anspruch 1 oder 2, **dadurch gekennzeichnet, daß** die Zusammensetzung A etwa 0,25 bis 2,5 Gew.-% eines Verdickungsmittels enthält.

4. Mittel nach einem oder mehreren der Ansprüche 1 bis 3, **dadurch gekennzeichnet, daß** die als Emulsion vorliegende Zusammensetzung B etwa 0,5 bis 10 Gew.-% mindestens eines C₁₀-C₂₄-Fettalkohols enthält.

5. Mittel nach einem oder mehreren der Ansprüche 1 bis 4, **dadurch gekennzeichnet, daß** die Zusammensetzung B etwa 0,25 bis 7,5 Gew.-% mindestens eines anionischen Tensids vom Sulfat-, Sulfonat-, Carboxylat- und/oder Alkylphosphat-Typ enthält.

6. Mittel nach einem oder mehreren der Ansprüche 1 bis 5, **dadurch gekennzeichnet, daß** die Zusammensetzung B etwa 0,1 bis 1 Gew.-% eines Parfums enthält.

7. Mittel nach einem oder mehreren der Ansprüche 1 bis 6, **dadurch gekennzeichnet, daß** die gebrauchsfertige Mischung aus den Zusammensetzungen A und B eine Viskosität zwischen etwa 100 und etwa 7500 mPa.s, gemessen bei 20°C im Brookfield-Rotationsviskosimeter, aufweist.

8. Verfahren zum Entfärben von gefärbten Haaren, **dadurch gekennzeichnet, daß** auf das zu entfärbende, nasse Haar eine Zusammensetzung aufgebracht wird, die einen pH-Wert von 2,5 bis 5,0 aufweist und durch Vermischen von zwei bis zur Anwendung räumlich getrennt voneinander gehaltenen Zusammensetzungen A und B erhalten wurde, wobei die Zusammensetzung A mindestens ein Reduktionsmittel in einer wäßrigen Grundlage enthält und einen alkalischen pH-Wert zwischen 7,5 und 12,5 aufweist, und die Zusammensetzung B als wäßrige Emulsion vorliegt, die mindestens einen fett- oder ölartigen Stoff, mindestens einen wasserlöslichen Emulgator und mindestens eine Parfumkomponente enthält und einen sauren pH-Wert von 1 bis 4 aufweist, nach etwa fünf- bis dreißigminütiger Einwirkungszeit aus dem Haar ausgespült, gegebenenfalls shampooniert und getrocknet wird.

9. Verfahren nach Anspruch 8, **dadurch gekennzeichnet, daß** das Haar im Anschluß an die Entfärbung mit einer 1- bis 6%-igen wäßrigen Wasserstoffperoxid-Zusammensetzung nachbehandelt wird.

## Claims

1. Composition for decoloring dyed hair, consisting of two Compositions A and B being kept separately until application, whereby the Composition A contains at least one reducing agent in an aqueous carrier and has an alkaline pH-value between 7.5 and 12.5, and the Composition B is present as an aqueous emulsion, containing at least one fatty or oily substance, at least one water-soluble emulsifier, and at least one perfume component, whereby a ready-to-use product with a pH-value between 2.5 and 5 is obtained upon admixing equal parts of both Compositions.

2. Composition according to claim 1, wherein the Composition A contains about 5 % to about 50 % by weight, calculated to its total composition, of hydroxymethane sulfinic acid and/or an alkali salt thereof.

3. Composition according to claim 1 or 2, wherein the Composition A contains about 0.25 % to 2.5 % by weight, calculated to its total composition, of a thickening agent.

4. Composition according to one or more of claims 1 to 3, wherein the Composition B contains about 0.5 % to 10 % by weight, calculated to its total composition, of at least one C₁₀-C₂₄-fatty alcohol.

5. Composition according to one or more of claims 1 to 4, wherein the Composition B contains about 0.25 % to 7.5 % by weight, calculated to its total composition, of at least one anionic surfactant of the sulfate, sulfonate, carboxylate and/or alkyl phosphate type.

6. Composition according to one or more of claims 1 to 5, wherein the Composition B contains about 0.1 % to about 1 % by weight, calculated to its total composition, of a perfume.

7. Composition according to one or more of claims 1 to 6, wherein the ready-to-use mixture of the Compositions A and B has a viscosity from about 100 to about 7.500 mPa.s, measured at 20°C in a Brookfield rotation viscosimeter.

8. Process for decoloring dyed hair, wherein a composition having a pH-value of 2.5 to 5.0, and being obtained by admixing two Compositions A and B, stored separately prior to application, is applied to wet hair, whereby the Composition A contains at least one reducing agent in an aqueous carrier and has an alkaline pH-value between 7.5 and 12.5, and the Composition B is present as an aqueous emulsion, containing at least one fatty or oily substance and at least one perfume component and having an acidic pH-value of 1 to 4, the mixture then being left to process for about five to thirty minutes, subsequently being rinsed out of the hair, the hair optionally being shampooed and dried.

9. Process according to claim 8, wherein the hair is treated with a 1 % to 6 % aqueous hydrogen peroxide composition subsequent to the decoloration.

## Revendications

1. Agent de décoloration de cheveux colorés, constitué de deux compositions A et B maintenues spatialement séparées l'une de l'autre jusqu'à leur utilisation, la composition A contenant au moins un agent de réduction dans une base aqueuse et présentant un pH alcalin d'une valeur comprise entre 7,5 et 12,5, la composition B se présentant sous la forme d'une émulsion aqueuse qui contient au moins une matière graisseuse ou huileuse, au moins un émulsifiant soluble dans l'eau et au moins un composant de parfum et présentant un pH acide d'une valeur comprise entre 1 et 4, tandis que lors du mélange de portions égales des deux compositions, on obtient un produit prêt à l'emploi dont la valeur du pH est comprise entre 2,5 et 5,0.

2. Agent selon la revendication 1, **caractérisé en ce que** la composition A contient d'environ 5 à environ 50 % en poids d'acide hydroxyméthane sulfinique et/ou d'un sel d'alcali de ce dernier.

3. Agent selon la revendication 1 ou 2, **caractérisé en ce que** la composition A contient environ 0,25 à environ 2,5 % en poids d'un agent épaississant.

4. Agent selon l'une ou plusieurs des revendications 1 à 3, **caractérisé en ce que** la composition B qui se présente sous forme d'émulsion contient d'environ 0,5 à environ 10 % en poids d'au moins un alcool gras en C₁₀ à C₂₄.

5. Agent selon l'une ou plusieurs des revendications 1 à 4, **caractérisé en ce que** la composition B contient d'environ 0,25 à 7,5 % en poids d'au moins un agent tensioactif anionique de type sulfate, sulfonate, carboxylate et/ou alkylphosphate.

6. Agent selon l'une ou plusieurs des revendications 1 à 5, **caractérisé en ce que** la composition B contient environ 0,1 à 1 % en poids d'un parfum.

7. Agent selon l'une ou plusieurs des revendications 1 à 6, **caractérisé en ce que** le mélange des compositions A et B prêt à l'emploi présente une viscosité, mesurée à 20°C dans un viscosimètre rotatif de Brookfield, qui est comprise entre environ 100 et environ 7 500 mPa.s.

8. Procédé pour décolorer des cheveux colorés, **caractérisé en ce que** l'on applique sur les cheveux à décolorer mouillés une composition qui présente un pH d'une valeur de 2,5 à 5,0 et qui a été obtenue par mélange de deux compositions A et B maintenues spatialement séparées l'une de l'autre jusqu'à leur utilisation, la composition A contenant au moins un agent réducteur dans une base aqueuse et présentant un pH alcalin d'une valeur comprise entre 7,5 et 12,5, la composition B se présentant sous la forme d'une émulsion aqueuse qui contient au moins une matière graisseuse ou huileuse, au moins un émulsifiant soluble dans l'eau et au moins un composant de parfum et présentant un pH acide d'une valeur comprise entre 1 et 4, les cheveux étant rincés après une durée d'action d'environ 5 à 30 minutes, éventuellement lavés au shampooing et séchés.

9. Procédé selon la revendication 8, **caractérisé en ce qu'**après la décoloration, les cheveux reçoivent un traitement final avec une composition aqueuse contenant de 1 à 6 % de peroxyde d'hydrogène.
